**Europäisches Patentamt**

**European Patent Office**

**Office européen des brevets**

(11) Publication number: **0 269 941**
**B1**

(12) **EUROPEAN PATENT SPECIFICATION**

(45) Date of publication of patent specification: **06.02.91**

(21) Application number: **87116889.4**

(22) Date of filing: **16.11.87**

(51) Int. Cl.⁵: **A 23 L 3/28, A 23 L 2/24, A 61 L 9/20**

(54) Fluid sterilizing process and device.

(30) Priority: **21.11.86 IT 6786486**

(43) Date of publication of application:
**08.06.88 Bulletin 88/23**

(45) Publication of the grant of the patent:
**06.02.91 Bulletin 91/06**

(84) Designated Contracting States:
**CH DE ES FR GB GR LI NL SE**

(56) References cited:
**DE-C- 885 344**
**US-A-2 586 625**
**US-A-4 534 282**

(73) Proprietor: **ULTRAVIOLET TECHNOLOGY
ITALIA S.r.l.
Corso Felice Cavallotti, 26
I-28100 Novara (IT)**

(72) Inventor: **Striglia, Silvio
Via Aronco, 28
I-28053 Castelletto Ticino (IT)**

(74) Representative: **Prato, Roberto et al
STUDIO TORTA Società Semplice Via Viotti 9
I-10121 Torino (IT)**

EP 0 269 941 B1

Courier Press, Leamington Spa, England.

## Description

The present invention relates to a fluid sterilizing process.

The effective sterilizing action of ultraviolet rays on fluids penetrated by the same is by now a well established fact.

As yet, however, this property of ultraviolet rays has generally been employed for sterilizing water or other alimentary fluids. In particular, devices are known for purifying and sterilizing drinks, such as water or similar fluids transparent to ultraviolet rays, on which the drink is fed through at least one vessel or tubular duct. The said duct is defined by a wall at least partly transparent to ultraviolet rays, and is set up in front of at least one source of the same. The ultraviolet rays produced by the said source thus pass through the said transparent wall and the drink, which is also transparent, so as to enable in-depth bombardment and effective sterilization of the drink particles.

A major drawback of purifying-sterilizing devices of the aforementioned type is that they are limited to sterilizing drinks, and cannot be employed for sterilizing other fluids, such as industrial drainage, usually containing highly contaminating organic substances which are in no way degraded.

The aim of the present invention is to provide a fluid sterilizing process designed to overcome the aforementioned drawback.

With this aim in view, according to the present invention, there is provided a fluid sterilizing process, characterised by the fact that it comprises stages consisting in:

—feeding fluid particles along a duct extending through an airtight casing and formed, at least partially, from material transparent to ultraviolet rays;

—feeding into the said casing a stream of a gaseous mixture containing oxygen;

—exhausting the said mixture from the said casing into the said duct; and

—simultaneously bombarding with ultraviolet rays the said mixture inside the said casing and the said particles flowing along the said duct; the said ultraviolet rays being produced by at least one source located outside the said duct and inside the said casing; the said source being designed to produce ultraviolet rays of such a wave length as to ozonize the said mixture inside the said casing.

According to the above process, subsequent to feeding the said ozonized mixture inside the fluid downstream from the said passage, the fluid presents, downstream from the ultraviolet ray process, an activated residue which is in no way toxic, by virtue of degrading rapidly in contact with the oxygen in the air, but which is extremely aggressive and capable of reducing by oxidation a wide variety of organic products.

Inside the said casing, the said duct preferably comprises a narrow passage defined by two walls, at least one of which is formed from material transparent to ultraviolet rays, and at least one of which is formed from unwettable material designed to produce, in the fluid flowing along the said passage, microvortices having a diameter substantially equal to the width of the said passage; the said mixture being fed inside the said duct downstream from the said passage; and the ultraviolet rays produced by the said source simultaneously bombarding both the said mixture inside the said casing, and the said particles as the swirl along with said passage and come into substantial contact with the said transparent wall.

In like manner, sterilization is achieved, not by the ultraviolet rays penetrating the fluid, but by means of superficial bombardment, thus substantially overcoming any functional limitation posed by non-transparent fluids. According to the present invention, there is also provided a fluid sterilizing device, characterised by the fact that it comprises an airtight casing; a duct for a stream of unsterilized fluid particles and extending through the said casing, the said duct being formed, at least partially, from material transparent to ultraviolet rays; first supply means for feeding a stream of a gaseous mixture containing oxygen inside the said casing; second supply means for feeding the said mixture from the said casing to a given point on the said duct; and at least one source of ultraviolet rays located inside the said casing, contacting the said mixture, and facing the said transparent portion of the said duct; the said source being designed to emit ultraviolet rays of such a wave length as to ozonize the said mixture.

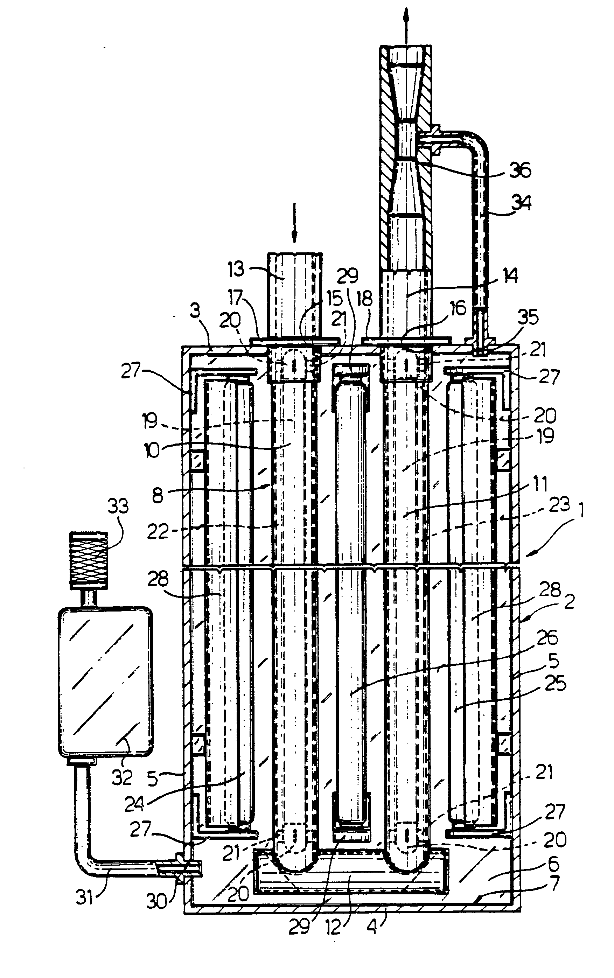

The present invention will be described with reference to the attached drawing showing an axial section of an embodiment of the same.

Number 1 in the attached drawing indicates a fluid purifying-sterilizing device, even for fluids impervious to ultraviolet rays, comprising an airtight outer casing 2 preferably formed from corrosion-proof metal, e.g. stainless steel, and in the form of a rectangular parallelepipedon.

The said casing 2 comprises a top wall 3, a bottom wall 4, a first pair of parallel side walls 5, and a second pair of parallel side walls 6 (only one of which is shown) perpendicular to walls 5.

Inside a chamber 7 defined by the said walls 3, 4, 5 and 6, there is housed a duct 8 for the said unsterilized fluid, which duct 8 may be of any shape, but, in the example shown, is substantially U-shaped with its concave side facing upwards. In more detail, the said duct 8 comprises a downward duct 10 and an upward duct 11 parallel with each other, formed from unwettable material transparent to ultraviolet rays, and connected at their respective bottom ends by a horizontal duct 12. The top ends of ducts 10 and 11 are engaged inside respective tubular ends of an outlet pipe 13 and inlet pipe 14 respectively, which pipes 13 and 14 extend through respective holes 15 and 16 formed through wall 3, and are connected to the said wall 3 via respective flanges 17 and 18.

Each of ducts 10 and 11 houses a tubular element 19 preferably formed from metal and closed at its opposite ends by plugs 20, each of which is secured to the inner surface of respective duct 10 or 11 by means of radial fins 21.

The said tubular elements 19 extend along substantially the entire length of respective ducts 10 and 11 and define, with the same, respective toroidal passages 22 and 23 along which the unsterilized fluid flows.

In the example shown, three fluorescent infrared lamps, 24, 25, 26, are mounted inside the said chamber 7. Lamps 24 and 25 are connected by means of respective end brackets 27 to respective walls 5, in a position adjacent to ducts 10 and 11 respectively, and present respective reflecting paraboloids 28 for concentrating the emitted rays on to ducts 10 and 11. Lamp 26, on the other hand, is located midway between ducts 10 and 11, and is supported by end brackets 29 connected to one of walls 6.

Each of lamps 24, 25 and 26 is designed to produce ultraviolet rays having wave lengths of 254 and 187 nm (nonometres), of which those having a wave length of 187 nm are known to be capable of ozonizing a gaseous mixture containing oxygen and, therefore, in the present case, a similar mixture contained inside airtight casing 2. Through one of walls 5, and close to the bottom end of the same, there is formed a hole 30 through which is fitted in sealed manner one end of an outlet pipe 31 on a compressor 32 having an inlet filter 33 and designed to feed a stream of a gaseous mixture containing oxygen, e.g. air, inside chamber 7. The said chamber 7 communicates with the outside atmosphere via an exhaust pipe 34 fitted in sealed manner through a hole 35 formed in wall 3. The said pipe 34 connects chamber 7 to a point on outlet pipe 14, which presents a section appropriately reduced to form a Venturi tube 36 constituting an ejector for the air inside chamber 7.

In actual use, the unsterilized fluid is fed into inlet pipe 13 by a supply network (not shown) and flows along duct 8 by successively flowing along passages 22, duct 12 and passage 23, as far as outlet pipe 14.

As it flows along toroidal passages 22 and 23, the unsterilized fluid assumes a swirling motion due to the fact that, as ducts 10 and 11 are formed from unwettable materials, such as TEFLON®, the fluid particles substantially contacting the inner surfaces of ducts 10 and 11 encounter substantially no frictional resistance and, therefore, tend to travel faster than the other particles. The size of the vortices formed inside passages 22 and 23 remains substantially constant for a given flow rate. Consequently, by selecting, for passages 22 and 23, a width approximately equal to the diameter of the vortices produced at the required flow rate, all the unsterilized fluid particles may be brought successively into contact with the inner surfaces of ducts 10 and 11, that is, into a position enabling the said particles to be sterilized by a superficial bombardment of ultraviolet rays emitted by lamps 24, 25 and 26.

As already stated, in addition to sterilizing the fluid flowing along duct 8, lamps 24, 25 and 26 simultaneously ozonize the air, or any other similar gaseous mixture containing oxygen, fed into casing 2 by compressor 32. The ozonized air is fed by ejector 36 into pipe 14 so as to enrich the sterilized fluid with an activated residue obtained using relatively little energy and capable of attacking and degrading a wide range of organic compounds contained in industrial drainage, such as phenols, and pesticides such as DDT.

The ozone contained in the sterilized fluid degrades rapidly in oxygen, in about 20 minutes, leaving no toxic residue and, therefore, rendering device 1 particularly suitable for processing drainage.

For example, according to a first variation (not shown), duct 8 may be formed without tubular elements 19 defining passages 22 and 23, and may consist of a coil or be of any appropriate shape other than the one described. Similarly, according to a further variation (not shown), passages 22 and 23 may be flat, instead of annular as in the example shown.

According to a further variation (not shown), tubular elements 19 may be formed from material designed to reflect ultraviolet rays, or, they too, from unwettable material transparent to ultraviolet rays.

According to a further variation (not shown), the ultraviolet lamps employed may differ in number and/or be positioned differently from those described herein.

According to a further variation (not shown), the means for supplying air or any other similar mixture inside casing 2 and/or the means for supplying the said mixture from casing 2 into the sterilized fluid stream may differ from those described herein.

Finally, according to a further variation (not shown), device 1 as described herein may constitute only one sterilizing unit on a system comprising a number of such units connected in various ways and housed either in respective casings 2 or in a single common airtight casing.

## Claims

1. A fluid sterilizing process, characterised by the fact that it comprises stages consisting in:
—feeding fluid particles along a duct extending through an airtight casing and formed, at least partially, from material transparent to ultraviolet rays;
—feeding into the said casing a stream of a gaseous mixture containing oxygen;
—exhausting the said mixture from the said casing into the said duct; and
—simultaneously bombarding with ultraviolet rays the said mixture inside the said casing and the said particles flowing along the said duct; the said ultraviolet rays being produced by at least one source located outside the said duct and inside the said casing; the said source being designed to produce ultraviolet rays of such a wave length as to ozonize the said mixture inside the said casing.

2. A process as claimed in Claim 1, characterized by the fact that, inside the said casing, the said duct comprises a narrow passage defined by two walls, at least one of which is formed from material transparent to ultraviolet rays, and at least one of which is formed from unwettable material designed to produce, in the fluid flowing along the said passage, microvortices having a diameter substantially equal to the width of the said passage; the said mixture being fed into the said duct downstream from the said passage; and the ultraviolet rays produced by the said source simultaneously bombarding both the said mixture inside the said casing, and the said particles as they swirl along the said passage and come into substantial contact with the said transparent wall.

3. A fluid sterilizing device, characterised by the fact that it comprises an airtight casing; a duct for a stream of unsterilized fluid particles and extending through the said casing, the said duct being formed, at least partially, from material transparent to ultraviolet rays; first supply means for feeding a stream of a gaseous mixture containing oxygen inside the said casing; second supply means for feeding the said mixture from the said casing to a given point on the said duct; and at least one source of ultraviolet rays located inside the said casing, contacting the said mixture, and facing the said transparent portion of the said duct; the said source being designed to emit ultraviolet rays of such a wave length as to ozonize the said mixture.

4. A device as claimed in Claim 3, characterised by the fact that the said duct comprises two opposite walls defining a relatively narrow passage, at least one of the said walls being formed, at least partially, from material transparent to ultraviolet rays, and at least one of the said walls being formed from unwettable material; the said second supply means being connected to a point on the said duct downstream from the said passage; and the width of the said passage being approximately the same size as the diameter of vortices induced in the said stream, in use, by the said wall of unwettable material.

5. A device as claimed in Claim 4, characterised by the fact that the said passage is toroidal; the said two walls being tubular and coaxial with each other.

6. A device as claimed in Claim 5, characterised by the fact that a first outer wall of the said tubular walls is formed from the said unwettable material transparent to ultraviolet rays; the said source of ultraviolet rays being located outside the said outer wall.

7. A device as claimed in any one of the foregoing Claims from 3 to 6, characterised by the fact that the said first supply means comprise a source of compressed air connected to the said casing.

8. A device as claimed in any one of the foregoing Claims from 2 to 7, characterised by the fact that the said second supply means comprise a Venturi ejector device.

9. A device as claimed in any one of the foregoing Claims from 3 to 8, characterised by the fact that the said source of ultraviolet rays is designed to emit ultraviolet rays having wave lengths of 254 and 187 nm.

**Patentansprüche**

1. Verfahren zur Sterilisation von Flüssigkeiten, gekennzeichnet durch Verfahrensschritte, die darin bestehen,
—dass die flüssigen Teilchen durch einen Kanal geführt werden, der sich durch ein luftdichtes Gehäuse erstreckt und der wenigstens teilweise aus einem für ultraviolette Strahlung durchsichtigen Material gebildet ist,
—das diesem Gehäuse ein Strom einer Sauerstoff enthaltenden Gasmischung zugeführt wird,
—dass ein Ausströmen dieser Mischung von diesem Gehäuse in diesen Kanal bewirkt wird, und
—dass diese Mischung innerhalb dieses Gehäuses und diese Teilchen, die durch diesen Kanal fliessen, gleichzeitig mit einer ultravioletten Strahlung bombardiert werden, wobei diese ultraviolette Strahlung durch mindestens eine Quelle erzeugt wird, die sich ausserhalb dieses Kanals und innerhalb dieses Gehäuses befindet, und wobei diese Quelle ausgebildet ist, um eine ultraviolette Strahlung mit einer derartigen Wellenlänge zu erzeugen, dass diese Mischung innerhalb dieses Gehäuses ozonisiert wird.

2. Verfahren nach Anspruch 1, dadurch gekennzeichnet, dass innerhalb dieses Gehäuses dieser Kanal einen engen Durchgang umfasst, der durch zwei Wände definiert ist, von denen mindestens eine aus einem für ultraviolette Strahlung durchsichtigen Material und mindestens eine von beiden aus einem unbenetzbaren Material gebildet ist, ausgestaltet, um in der Flüssigkeit, die durch diesen Durchgang fliesst, Mikrowirbel zu erzeugen, die im wesentlichen denselben Durchmesser wie die Breite dieses Durchgangs haben, wobei diese Mischung stromabwärts von diesem Durchgang in diesen Kanal eingeführt wird, und dass die von dieser Quelle erzeugte ultraviolette Strahlung gleichzeitig sowohl diese Mischung innerhalb dieses Gehäuses als auch diese Teilchen bombardiert, wenn sie durch diesen Durchgang wirbeln und im wesentlichen in Kontakt mit dieser durchsichtigen Wand kommen.

3. Vorrichtung zur Sterilisation einer Flüssigkeit, dadurch gekennzeichnet, dass sie ein luftdichtes Gehäuse und einen sich durch dieses Gehäuse erstreckenden Kanal für einen Strom von nichtsterilisierten flüssigen Teilchen umfasst, wobei dieser Kanal wenigstens teilweise aus einem für ultraviolette Strahlung durchsichtigen Material gebildet ist, sowie erste Zufuhrmittel zum Einführen eines Stroms einer Sauerstoff enthaltenden Gasmischung in dieses Gehäuse und zweite Zufuhrmittel umfasst, um diese Mischung von diesem Gehäuse zu einem vorgegebenen Punkt dieses Kanals zu führen, und dass sich innerhalb dieses Gehäuses und gegenüber diesem durchsichtigen Teil des Kanals wenigstens eine Quelle für Ultraviolett-Strahlung befindet, die diese Mischung berührt, wobei diese Quelle ausgebildet ist, um eine ultraviolette Strahlung mit einer derartigen Wellenlänge zu erzeugen, dass diese Mischung ozonisiert wird.

4. Vorrichtung nach Anspruch 3, dadurch gekennzeichnet, dass dieser Kanal zwei gegenüberliegende Wände umfasst, die einen relativ engen Durchgang definieren, wobei mindestens eine dieser Wände wenigstens teilweise aus einem für ultraviolette Strahlung durchsichtigen Material und mindestens eine dieser Wände aus einem nichtbenetzbaren Material gebildet ist, wobei die zweiten Zufuhrmittel an einen Punkt dieses Kanals stromabwärts von diesem Durchgang angeschlossen sind, und wobei die Breite dieses Durchgangs ungefähr gleich wie der Durchmesser der Wirbel ist, die in diesem Strom, in Gebrauch, durch diese nichtbenetzbare Wand induziert werden.

5. Vorrichtung nach Anspruch 4, dadurch gekennzeichnet, dass dieser Durchgang eine toroidale Form aufweist, wobei diese zwei Wände röhrchenförmig und gegeneinander koaxial angeordnet sind.

6. Vorrichtung nach Anspruch 5, dadurch gekennzeichnet, dass von diesen röhrchenförmigen Wänden eine erste Aussenwand aus diesem nichtbenetzbaren, für Ultraviolett-Strahlung durchsichtigen Material gebildet ist, wobei die Ultraviolett-Strahlungsquelle ausserhalb dieser Aussenwand angeordnet ist.

7. Vorrichtung nach einem der vorhergehenden Ansprüche 3 bis 6, dadurch gekennzeichnet, dass die ersten Zufuhrmittel eine an dieses Gehäuse angeschlossene Quelle von komprimierter Luft umfassen.

8. Vorrichtung nach einem der vorhergehenden Ansprüche 2 bis 7, dadurch gekennzeichnet, dass diese zweiten Zufuhrmittel eine Venturi-Düsen-Vorrichtung umfassen.

9. Vorrichtung nach einem der vorhergehenden Ansprüche 3 bis 8, dadurch gekennzeichnet, dass diese Ultraviolett-Strahlungsquelle ausgebildet ist, um eine ultraviolette Strahlung mit einer Wellenlänge von 254 und 187 nm auszustrahlen.

**Revendications**

1. Un procédé de stérilisation de fluide, caractérisé en ce qu'il comporte les étapes comprenant:
—l'alimentation de particules de fluide dans un conduit s'étendant au travers d'une enveloppe étanche à l'air et formée, au moins en partie, d'un matériau perméable aux rayons ultraviolets,
—l'alimentation dans ladite enveloppe d'un courant de mélange gazeux contenant de l'oxygène,
—l'évacuation dudit mélange de l'enveloppe dans ledit conduite, et
—en même temps, le bombardement aux rayons ultraviolets dudit mélange à l'intérieur de ladite enveloppe et l'écoulement desdites particules le long dudit conduit, lesdits rayons ultraviolets étant produits par au moins une source située à l'extérieur dudit conduit et à l'intérieur de ladite enveloppe, ladite source étant agencée pour émettre des rayons ultraviolets d'une longueur d'onde permettant d'ozoniser ledit mélange à l'intérieur de ladite enveloppe.

2. Un procédé selon la revendication 1, caractérisé en ce qu'à l'intérieur de ladite enveloppe, ledit conduit comprend un passage étroit délimité par deux parois, au moins l'une d'elles étant formée à partir d'un matériau perméable aux rayons ultraviolets et au moins l'une d'elles étant formée d'un matériau non-mouillable, agencé pour produire, dans le courant de fluide au travers dudit passage, des micro-tourbillons d'un diamètre sensiblement égal à la largeur dudit passage, ledit mélange étant acheminé dans ledit conduit en aval dudit passage, et les rayons ultraviolets produits par ladite source bombardant en même temps, à la fois ledit mélange à l'intérieur de ladite enveloppe, et lesdites particules pendant qu'elles tourbillonnent dans ledit passage et viennent sensiblement en contact avec ladite paroi perméable.

3. Un dispositif de stérilisation de fluide, caractérisé en ce qu'il comprend une enveloppe étanche à l'air, un conduit par un courant de particules de fluide non stérilisées et s'étendant au travers de ladite enveloppe, ledit conduit étant formé, au moins partiellement, d'un matériau perméable aux rayons ultraviolets, des premiers moyens d'alimentation d'un courant de mélange gazeux contenant de l'oxygène à l'intérieur de ladite enveloppe, des seconds moyens d'alimentation pour alimenter ledit mélange à partir de ladite enveloppe jusqu'à un point donné dudit conduit, et au moins une source de rayons ultraviolets située à l'intérieur de ladite enveloppe, en contact avec ledit mélange et tournée vers ladite partie perméable dudit conduit, ladite source étant agencée pour émettre des rayons ultraviolets d'une longueur d'onde telle qu'elle assure l'ozonisation dudit mélange.

4. Un dispositif selon la revendication 3, caractérisé en ce que ledit conduit comprend deux parois opposées délimitant un passage relativement étroit, au moins l'une desdites parois étant formée, au moins en partie, d'un matériau perméable aux rayons ultraviolets, et au moins une desdites parois étant formée à partir d'un matériau non-mouillable, lesdits seconds moyens d'alimentation étant reliés à un point dudit conduit situé en aval dudit passage et la largeur dudit passage étant approximativement de même dimension que le diamètre des tourbillons produits dans ledit courant, en service, par ladite paroi de matériau non-mouillable.

5. Un dispositif selon la revendication 4, caractérisé en ce que ledit passage est toroïdal, lesdites deux parois étant tubulaires et coaxiales l'une par rapport à l'autre.

6. Un dispositif selon la revendication 5, caractérisé en ce que la première paroi extérieure desdites parois tubulaires est formée à partir d'un matériau non-mouillable, perméable aux rayons ultraviolets, ladite source de rayons ultraviolets étant située à l'extérieur de ladite paroi extérieure.

7. Un dispositif selon l'une quelconque des précédentes revendications 3 à 6, caractérisé en ce que lesdits premiers moyens d'alimentation comprennent une source d'air comprimé reliée à ladite enveloppe.

8. Un dispositif selon l'une quelconque des précédentes revendications 2 à 7, caractérisé en ce que lesdits seconds moyens d'alimentation comprennent un dispositif à éjecteur de Venturi.

9. Un dispositif selon l'une quelconque des précédentes revendications 3 à 8, caractérisé en ce que ladite source de rayons ultraviolets est prévue pour émettre des rayons ultraviolets d'une longueur d'onde de 254 et de 187 nm.